# EUROPEAN PATENT SPECIFICATION

(11) **EP 1 547 573 B1**
(45) Date of publication and mention of the grant of the patent: **14.02.2007**
(21) Application number: 04257735.3
(22) Date of filing: 13.12.2004
(51) Int. Cl.: A61K 8/81, A61Q 3/02

(54) **Aqueous nail varnish with fast drying properties**
Schnelltrocknende wässrige Nagellackzusammensetzung
Composition aqueuse de vernis à ongles à séchage rapide

(30) Priority: 22.12.2003 EP 03293282
(43) Date of publication of application: 29.06.2005
(73) Proprietor: ROHM AND HAAS COMPANY, Philadelphia, Pennsylvania 19106-2399 (US)
(72) Inventor: Duccini, Yves, Ville Soleoda 06250 Mougins (FR); Sanfilippo, Angelo, 06210 Mandelieu (FR); Ugazio, Stephane Pierre Jean, 06530 Carbris (FR)
(74) Representative: Buckley, Guy Julian

(56) References cited:
- EP-A- 0 752 244
- EP-A- 0 882 750
- US-A- 5 922 398
- US-B1- 6 319 977

## Description

The present invention relates to a method for coating human nails with a polymer film. The method employs an aqueous composition which has fast drying properties.

U.S. Pat. No. 5,965,111 discloses a fast drying water-based nail varnish formulation. However, this formulation contains at least 20% by weight of a volatile organic solvent.

EP-A-0 752 244 discloses said varnish compositions comprising a film forming polymer which contains carboxylic acid groups or amine groups.

EP-A-0 882 750 discloses the use of polymers having acid and amine functionalities in said varnish compositions.

The problem addressed by this invention is the need for a fast drying aqueous nail varnish that contains lower amounts of volatile organic solvents, especially those known to be detrimental to the environment and/or human health, commonly known as "VOC" solvents.

### STATEMENT OF THE INVENTION

The present invention is as set out in the accompanying claims.

The present invention is directed to a method of coating human nails with a polymer film; said method comprising steps of: (a) applying to the human nails a composition comprising: (i) a polymer binder having pendant amine functional groups, carboxylic acid functional groups and a T_{g} greater than 0°C; (ii) an amount of volatile base sufficient to raise pH of the composition to a point where essentially all of the amine functional groups are in a non-ionic state; and (iii) water; wherein the composition contains less than 20% of organic solvents; and (b) allowing the composition to dry until the polymer film has formed.

The present invention is further directed to an aqueous composition suitable for use as a nail polish comprising: (a) a polymer binder having pendant amine functional groups, carboxylic acid functional groups and a T_{g} greater than 0°C; (b) an amount of volatile base sufficient to raise pH of the composition to a point where all of the amine functional groups are in a non-ionic state; (c) perfume; and (d) water; wherein the composition contains less than 20% of organic solvents and less than 10% inorganic material.

### DETAILED DESCRIPTION OF THE INVENTION

The term "human nail" refers to a fingernail or toenail of a human being. All percentages and ppm values are on the basis of total weight of the composition, unless otherwise indicated. The term "acrylic polymers" refers to polymers of acrylic acid (AA), methacrylic acid (MAA) and their esters, and copolymers made from monomer mixtures predominantly comprising the aforementioned monomers. Esters of AA and MAA include, but are not limited to, methyl methacrylate (MMA), ethyl methacrylate (EMA), butyl methacrylate (BMA), hydroxyethyl methacrylate (HEMA), methyl acrylate (MA), ethyl acrylate (EA), butyl acrylate (BA), and hydroxyethyl acrylate (HEA), as well as other alkyl, hydroxyalkyl and aminoalkyl esters of AA or MAA. Acrylic polymers also may contain monomer units derived from other ethylenically unsaturated monomers, e.g., styrene or substituted styrenes; other α,β-unsaturated carboxylic acids, esters and amides; vinyl esters or halides; etc. Preferably, an acrylic polymer contains less than 30% of these other monomer units, more preferably less than 10%, and most preferably the acrylic polymers are substantially free of monomer units other than those of AA, MA and their esters. The term "inorganic" refers to materials that do not contain carbon, with the exceptions that metal salts containing carbonate are considered to be inorganic, and water and ammonia are not considered to be inorganic.

In one embodiment of the invention the pendant amine functional groups and the carboxylic acid groups are part of the same polymer, introduced by copolymerizing amine-substituted monomers and carboxylic acid monomers. In another embodiment of the invention, the pendant amine functional groups and the carboxylic acid groups are not part of the same polymer, but instead, two separate polymers are blended to produce the final binder. One of the polymers contains pendant amines, and the other contains carboxylic acid groups. In this embodiment, the aqueous composition comprises: (a) a polymer binder comprising: (i) a polymer having pendant amine functional groups and a T_{g} greater than 0°C; and (ii) a polymer having carboxylic acid functional groups and a T_{g} greater than 0°C; (b) an amount of volatile base sufficient to raise pH of the composition to a point where all of the amine functional groups are in a non-ionic state; and (c) water; wherein the composition contains less than 20% of organic solvents.

The polymer binder having pendant amine functional groups useful in the present invention includes all amine-functionalized polymers having a T_{g} greater than 0°C, such that aqueous coating compositions containing such polymers, in the presence of a solvent, e.g., a coalescent, are capable of film formation at ambient temperatures or greater. Polymers having pendant amine functional groups, in particular amine-functionalized latexes are described, for example, in U.S. Pat. No. 5,922,398, and references cited therein. The amount of amine present in the polymer binder having pendant amine functional groups will vary depending on the amine functionality and the method of preparation, but preferably the total weight of the amine-containing monomer units comprises 0.5-10% of the total weight of polymer in the composition, more preferably from 0.5 to 5%, more preferably from 0.5 to 3%, and most preferably from 1 to 3%.

In the aqueous compositions of the present invention, all of the amine-functionalized latex is maintained in a deprotonated state by raising the pH of the composition to a pH in the range of 7.5 - 11, preferably 9 - 10.5, and most preferably 9 - 10. This means all of the amine groups in the amine-functionalized latex are in a deprotonated state. The pH can be raised by adding a base such as: ammonia; an alkali metal hydroxide, such as sodium hydroxide; or morpholine or other lower alkyl amines, such as 2-methylaminoethanol, 2-dimethylaminoethanol, N-methylmorpholine and ethylenediamine. Volatile bases, such as ammonia, or a mixture of volatile bases and nonvolatile bases, such as sodium hydroxide, are preferred; and ammonia is most preferred. This deprotonation of the amine functional groups helps to preserve the colloidal stability of the composition.

The aqueous compositions of the present invention also contain an acid-containing latex, or the amine-containing latex may optionally also contain carboxylic acid functional groups; that is, the polymer in the latex has pendant carboxylic acid groups. The addition of acid functional groups is believed, without reliance thereon, to enhance the stability of the composition. Acid-containing latexes are well known to those skilled in the art, and their preparation will not be further discussed herein.

The amount of acid present in the acid-containing latex or the acid/amine-containing latex will vary, depending on the acid monomer utilized, and the method of preparation, but preferably, the total weight of the carboxylic acid-containing monomer units comprises from 0.5-10 wt% of the total weight of polymer in the composition, more preferably from 1-5 wt%, and most preferably from 1-3 wt%. Acid/amine-containing latexes are described, for example, in U.S. Pat. Nos. 3,404,114 and 4,760,110. The amount of acid present in the acid/containing latex or the acid/amine-containing latex is also a function of the amount of amine. Preferably, the weight ratio of amine-containing monomer units to carboxylic acid-containing monomer units in the polymer is at least 3:1, more preferably at least 5:1, and most preferably at least 10:1. Preferably this ratio is less than 50:1.

Preferably, the polymer binder used in the present invention contains an acrylic polymer. Other suitable polymers are made by addition polymerization of vinyl monomers, e.g., monomers selected from among styrenes; crotonic, itaconic, fumaric and maleic acids and their esters; acrylamides; butadiene; vinyl esters; vinyl halides; vinylidene halides; N-vinylpyrrolidone; sodium vinyl sulfonate; acrolein and methacrolein. Suitable ethylenically-unsaturated amine-containing monomers are described, for example, in U.S. Pat. No. 6,013,721, and include 2-oxazolidinylethyl acrylate and methacrylate, and alkylaminoalkyl estes of α,β-unsaturated carboxylic acids, especially 2-dimethylaminoethyl acrylate and methacrylate. Amine-containing polymers also can be prepared by polymerization or copolymerization of ethyleneimine or propyleneimine.

In one embodiment of the invention, in addition to the polymeric binder having pendant amine functional groups and carboxylic acid functional groups, the composition contains at least one polyurethane binder. The polyurethane binder comprises, for example, a polyether polyurethane, a polyester polyurethane, or a combination thereof. The polyurethane binder may be aliphatic, aromatic, or a combination thereof. Preferably, the polyurethane binder is present in an amount no more than 50%, based on weight of the polyurethane solids as a percentage of the total polymer weight in the composition, more preferably no more than 30%, and most preferably no more than 20%.

Preferably, the polymer binder content of the composition, measured as dry polymer, is from 20-60%, more preferably from 30-50%. Preferably, the drying time, i.e., the time at which the film is not sticky to a light touch, is less than five minutes for a film thickness of 75 to 100 microns, more preferably from fifteen seconds to three minutes.

Surfactants are commonly used in emulsion or dispersion polymerization to provide stability, as well as to control particle size. Surfactants can also provide dispersibility for water-reducible resins. Conventional surfactants include anionic or nonionic emulsifiers or combinations thereof. Typical anionic emulsifiers include but are not limited to: alkali or ammonium alkyl sulfates, alkyl sulfonates, salts of fatty acids, esters of sulfosuccinic acid salts, alkyl diphenylether disulfonates, and salts or free acids of complex organic phosphate esters. Typical nonionic emulsifiers include but are not limited to: polyethers, e.g. ethylene oxide and propylene oxide condensates which include straight and branched chain alkyl and alkylaryl polyethylene glycol and polypropylene glycol ethers and thioethers, alkyl phenoxypoly(ethyleneoxy) ethanols having alkyl groups containing from about 7 to about 18 carbon atoms and having from about 4 to about 100 ethyleneoxy units, and polyoxyalkylene derivatives of hexitol, including sorbitans, sorbides, mannitans, and mannides. Surfactants may be employed in the compositions of the present invention at levels of 0.05 - 1 wt% or greater, based on the total weight of the final composition.

The aqueous compositions of the present invention may optionally contain additional components including but not limited to: thickeners; rheology modifiers; dyes; sequestering agents; biocides; dispersants; colorants such as the typical organic dyes and inorganic pigments used in the cosmetics and paint industries; plasticizers; adhesion promoters; coalescents; wetting agents; waxes; surfactants; slip additives; crosslinking agents; defoamers; preservatives; perfumes (at 0.05% to 1%); freeze/thaw protectors; and alkali or water soluble polymers, including other binders that can increase film hardness, adhesion and water resistance, such as polyurethanes or classical- or core-shell-type latexes. In one embodiment of the invention, the aqueous composition contains 1-10% of a wax, more preferably from 1.5-8%, and most preferably from 2-6%. Polyolefin waxes are preferred.

Preferably, the aqueous compositions contain less than 15% of inorganic material, more preferably less than 10%. In one embodiment of the invention, they contain less than 7% inorganic material. In another embodiment of the invention, the aqueous composition is substantially free of inorganic material; preferably in this embodiment, the nail varnish is clear and colorless. Inorganic materials that may be used in the composition include, for example, inorganic pigments and colored inorganic particles.

The aqueous compositions used in the present invention contain less than 20% of organic solvents. Preferably the compositions contain less than 15% of organic solvents, more preferably less than 12%, and most preferably less than 10%. Preferably the aqueous compositions contain less than 5% of VOC solvents, more preferably less than 2%, and most preferably the compositions are substantially free of VOC solvents. VOC solvents are those organic solvents that have non-negligible atmospheric photochemical reactivity. The term "VOC solvent" is defined in readily accessible environmental regulations in most jurisdictions.

As this nail varnish is aqueous, it can be prepared easily in situ (e.g., in shops) by adding to a partially formulated aqueous polymer binder any colorant or pigments or perfumes preferred by the customer. Hence a customer could establish the color and fragrance of the nail varnish, and the vendor could prepare the desired nail varnish accordingly from a base formulation not containing these ingredients.

### EXAMPLES

### Example 1: Comparison of a Fast Drying Aqueous Nail Varnish with a Conventional Aqueous System

The following formulations were prepared:

| | Fast-Dry | Conventional |
|---|---|---|
| Acrylic Binder A¹ | 177 | ---- |
| Acrylic Binder B² | ---- | 177 |
| Defoamer³ | 0.70 | 0.70 |
| Solvent⁴ | 8.42 | 8.42 |
| Thickener⁵ | 0.40 | 0.40 |
| Wetting Agent⁶ | 6.20 | 6.20 |
| TOTAL | 192.7 | 192.7 |

| | | |
|---|---|---|
| Notes 1. Acrylic Binder A contains: a 50% solids aqueous binder containing 97.6 parts of a 67:31:2, MMA/BA/MAA polymer; 0.3 parts 25% ammonia; 2.3 parts of a 28% solids polymer latex containing a homopolymer of 2-oxazolidinylethyl methacrylate; with a pH of 9.5. | | |
| 2. Acrylic Binder B was a 50% solids aqueous binder containing a 67:31:2, MMA/BA/MAA polymer; with a pH of 9.5. | | |
| 3. The defoamer was Nopco 8034 (available from Cognis Co. in France), a sulfated castor oil. | | |
| 4. The solvent was TEXANOL (2,2,4-trimethyl-1,3-pentanediol, mono-isobutyrate ester; available from Eastman Co., Kingsport TN) | | |
| 5. The thickener was QR2020 (available from Rohm and Haas Company, Philadelphia, PA), a polyurethane thickener | | |
| 6. The wetting agent was TEGO 450 (available as a 1% aqueous solution from Degussa in Germany), a polyether siloxane copolymer | | |

The formulations were applied to a glass substrate using an applicator which produced a wet film of controlled thickness. Drying time was evaluated by applying a finger to the film with light pressure, with the drying time being the time in minutes at which a finger no longer stuck to the film. The results are shown below.

| Wet Film Thickness | Fast-Dry | Conventional |
|---|---|---|
| 100 µm | 3 | 10 |
| 175 µm | 12 | 21 |

### Example 2: Nail Varnishes Containing Perfume, and Peel Test

The following nail varnishes were formulated:

| | Varnish 1 | Varnish 2 |
|---|---|---|
| Acid Binder¹ | 856.8 | 856.8 |
| Defoamer² | 1.8 | 1.8 |
| Ammonia, 28% | 11 | 11 |
| Amine Binder³ | 20.7 | 20.7 |
| Solvent⁴ | 92.5 | 92.5 |
| Water | 18 | 18 |
| Thickener⁵ | 2.5 | 2.5 |
| TOTAL | 1003.3 | 1003.3 |
| Perfume A⁶ | 1% | ---- |
| Perfume B⁷ | ---- | 1% |

| | | |
|---|---|---|
| Notes 1. The Acid Binder was a 50% solids aqueous binder containing a 67:31:2, MMA/BA/MAA polymer; with a pH of 9.5. | | |
| 2. The defoamer was Nopco NDW (available from Cognis Co. in France), a sulfated castor oil | | |
| 3. The Amine Binder was a 28% solids polymer latex containing a homopolymer of 2-oxazolidinylethyl methacrylate; with a pH of 9.5. | | |
| 4. The solvent was TEXANOL (2,2,4-trimethyl-1,3-pentanediol, mono-isobutyrate ester; available from Eastman Co., Kingsport TN) | | |
| 5. The thickener was RM5 (available from Rohm and Haas Co., Philadelphia PA), a polyacrylic acid thickener. | | |
| 6. Perfume A was a "framboise perfume" from Mane SA in France | | |
| 7. Perfume B was a "pomme perfume" from Mane SA in France | | |

After applying these formulations to nails, the odor of the perfume was still perceptible. After one hour at room temperature the film was easily removed by peeling.

### Example 3: Comparison of two Fast Drying Aqueous Nail Varnishes with a Conventional Aqueous System

The following formulations were prepared:

| | Fast-Dry 1 | Fast-Dry 2 | Conventional |
|---|---|---|---|
| Acrylic Binder A¹ | 900 | ---- | ---- |
| Acrylic Binder B² | ---- | 900 | ---- |
| Acrylic Binder C³ | ---- | ---- | 900 |
| Defoamer⁴ | 2 | 2 | 2 |
| Solvent⁵ | 60 | 30 | 60 |
| Thickener⁶ | 5 | 5 | 5 |
| Wax⁷ | 50 | 50 | 50 |
| Wetting Agent⁸ | 3 | 3 | 3 |
| Colorant⁹ | 50 | 50 | 50 |
| Water | 55 | 55 | 55 |
| Perfume¹⁰ | 2 | 2 | 2 |
| TOTAL | 1127 | 1097 | 1127 |

| | | | |
|---|---|---|---|
| Notes 1. Binder A was a 50% solids aqueous binder containing 97.6 parts of a 67:31:2, MMA/BA/MAA polymer; 0.3 parts of 25% ammonia; and 2.3 parts of a homopolymer of 2-oxazolidinylethyl methacrylate; with a pH of 9.5. | | | |
| 2. Binder B was a 50% solids aqueous binder containing 97.6 parts of a 53:45:2, MMA/BA/MAA polymer; 0.3 parts of 25% ammonia; and 2.3 parts of a homopolymer of 2-oxazolidinylethyl methacrylate; with a pH of 9.5. | | | |
| 3. Binder C was a 50% solids aqueous binder containing a 67:31:2, MMA/BA/MAA polymer; with a pH of 9.5. | | | |
| 4. The defoamer was Nopco NDW (available from Cognis Co. in France), a sulfated castor oil | | | |
| 5. The solvent was TEXANOL (2,2,4-trimethyl-1,3-pentanediol, mono-isobutyrate ester; available from Eastman Co., Kingsport TN) | | | |
| 6. The thickener was RM2020 (available from Rohm and Haas Co., Philadelphia, PA), a polyurethane thickener | | | |
| 7. The wax was Ultralube E340 (available from Keim-Additec, Surface GmBH), a polyethylene wax. | | | |
| 8. The wetting agent was TEGO 450 (available from Tego Service GmbH, Germany), a polyether siloxane copolymer | | | |
| 9. The coloring agent was Colter Colourant VS OPE free (available from CPS Color, the Netherlands) | | | |
| 10. The perfume was a "framboise perfume" (available from Mane SA, France) | | | |

The solids content of Fast-Dry 1, Fast-Dry 2 and the Conventional formulation was 41%, and the MFT film forming temperature for each was 10°C. When applied at a film thickness of 75 microns, Fast-Dry 1 and Fast-Dry 2 had a drying time of one minute, while the Conventional formulation had a drying time of five minutes.

### Example 4: Effect of Addition of Polyurethane on the Film Adhesion to the Nail.

The following formulations were prepared:

| | Formulation 1 | Formulation 2 |
|---|---|---|
| Acid Binder¹ | 28.1 | 28.1 |
| Defoamer² | 0.06 | 0.06 |
| Amine Binder³ | 0.38 | 0.38 |
| Solvent⁴ | 1.52 | 1.52 |
| Polyurethane⁵ | 0 | 7 |
| TOTAL | 30.06 | 37.06 |

| | | |
|---|---|---|
| Notes 1. The Acid Binder was a 50% solids aqueous binder containing a 67:31:2, MMA/BA/MAA polymer; and neutralized using ammonia at pH of 9.6. | | |
| 2. The defoamer was Nopco NDW (available from Cognis Co. in France), a sulfated castor oil. | | |
| 3. The Amine Binder was a 28% solids polymer latex containing a homopolymer of 2-oxazolidinylethyl methacrylate; with a pH of 9.5. | | |
| 4. The solvent was TEXANOL (2,2,4-trimethyl-1,3-pentanediol, mono-isobutyrate ester; available from Eastman Co., Kingsport TN) | | |
| 5. The polyurethane dispersion was Vithane™ 3936 an aliphatic aqueous polyurethane topcoat, 40% solids (available from Rohm and Haas Co., Philadelphia, PA) | | |

The formulations were applied to a glass substrate using an applicator which produced a wet film of controlled thickness. Drying time was evaluated by applying a finger to the film with light pressure, with the drying time being the time in minutes at which a finger no longer stuck to the film. The results are shown below.

| Wet Film Thickness | Formulation 1 | Formulation 2 |
|---|---|---|
| 100 µm | 4 | 6 |

The formulations were then applied on the fingernails of a panel of four people. After 1 hour, the panelists tried to remove the coating by simply scratching the nails. Clearly the film coming from Formulation 1 could easily be removed. However, using Formulation 2, the adhesion was improved by the polyurethane so that removability was prevented. Fast drying properties were maintained in the presence of the polyurethane, as shown by the drying times listed above.

## Claims

1. A method of coating human nails with a polymer film; said method comprising steps of:
(a) applying to the human nails a composition comprising: (i) a polymer binder having pendant amine functional groups, carboxylic acid functional groups and a T_{g} greater than 0°C, wherein the pendant amine functional groups and the carboxylic groups are part of the same polymer, introduceable by copolymerizing amine-substituted monomers and carboxylic acid monomers, or a polymer binder comprising a polymer having carboxylic acid functional groups and a T_{g} greater than 0°C and a polymer having pendant amine functional groups and a T_{g} greater than 0°C; (ii) an amount of volatile base sufficient to raise pH of the composition to a point where all of the amine functional groups are in a non-ionic state; and (iii) water; wherein the composition contains less than 20% of organic solvents; and
(b) allowing the composition to dry until the polymer film has formed.

2. The method of claim 1 in which the aqueous composition contains less than 12% of organic solvents, less than 5% of VOG solvents, and less than 10% inorganic material.

3. The method of claim 2, further comprising from 1-10% of a wax.

4. The method of claim 1 in which said polymer binder comprises:
(a) a polymer having pendant amine functional groups and a T_{g} greater than 0°C; and
(b) a polymer having carboxylic acid functional groups and a T_{g} greater than 0°C.

5. The method of claim 4, in which the aqueous composition contains less than 12% of organic solvents, less than 5% of VOC solvents, and further comprising from 1-10% of a wax.

6. An aqueous composition suitable for use as a nail polish comprising:
(a) a polymer binder having pendant amine functional groups, carboxylic acid functional groups and a T_{g} greater than 0°C , wherein the pendant amine functional groups and the carboxylic groups are part of the same polymer, by copolymerizing amine-substituted monomers and carboxylic acid monomers, or a polymer binder comprising polymer having carboxylic acid functional groups and a T_{g} greater than 0°C and a polymer having pendant amine functional groups and a T_{g} greater than 0°C;
(b) an amount of volatile base sufficient to raise pH of the composition to a point where all of the amine functional groups are in a non-ionic state;
(c) perfume; and
(d) water;
wherein the composition contains less than 20% of organic solvents and less than 10% inorganic material which is other than ammonia and water.

7. The aqueous composition of claim 6 in which the composition contains less than 12% of organic solvents and less than 5% of VOC solvents.

8. The aqueous composition of claim 7, further comprising at least one polyurethane binder.

9. The aqueous composition of claim 6 in which said polymer binder comprises:
(a) a polymer having pendant amine functional groups and a T_{g} greater than 0°C; and
(b) a polymer having carboxylic acid functional groups and a T_{g} greater than 0°C.

10. The aqueous composition of claim 9, further comprising at least one polyurethane binder.

## Patentansprüche

1. Verfahren zum Beschichten menschlicher Nägel mit einem Polymerfilm, das Verfahren umfassend die Schritte
(a) des Aufbringens einer Zusammensetzung, umfassend (i) ein Polymerbindemittel mit seitenständigen Amin-funktionellen Gruppen, Carbonsäure-funktionellen Gruppen und einer T_{g} von mehr als 0°C, wobei die seitenständigen Amin-funktionellen Gruppen und die Carbonsäuregruppen Teil des gleichen Polymers sind, einführbar durch das Copolymerisieren Amin-substituierter Monomere und Carbonsäure-Monomere, oder ein Polymerbindemittel, umfassend ein Polymer mit Carbonsäure-funktionellen Gruppen und einer T_{g} von mehr als 0°C und ein Polymer mit seitenständigen Amin-funktionellen Gruppen und einer T_{g} von mehr als 0°C, (ii) eine Menge flüchtiger Base, ausreichend, um den pH der Zusammensetzung auf einen Punkt zu heben, an dem alle Amin-funktionellen Gruppen in einem nicht-ionischen Zustand vorliegen, und (iii) Wasser, wobei die Zusammensetzung weniger als 20% organische Lösungsmittel enthält, auf die menschlichen Nägel und
(b) des Erlaubens, dass die Zusammensetzung trocknet, bis sich der Polymerfilm gebildet hat.

2. Verfahren nach Anspruch 1, in welchem die wässrige Zusammensetzung weniger als 12% organische Lösungsmittel, weniger als 5% VOC-Lösungsmittel und weniger als 10% anorganisches Material enthält.

3. Verfahren nach Anspruch 2, weiter umfassend von 1 bis 10% eines Wachses.

4. Verfahren nach Anspruch 1, in welchem das Polymerbindemittel umfasst:
(a) ein Polymer mit seitenständigen Amin-funktionellen Gruppen und einer T_{g} von mehr als 0°C und
(b) ein Polymer mit Carbonsäure-funktionellen Gruppen und einer T_{g} von mehr als 0°C.

5. Verfahren nach Anspruch 4, in welchem die wässrige Zusammensetzung weniger als 12% organische Lösungsmittel, weniger als 5% VOC-Lösungsmittel enthält und weiter von 1 bis 10% eines Waches umfasst.

6. Wässrige Zusammensetzung, geeignet zur Verwendung als eine Nagelpolitur, umfassend:
(a) ein Polymerbindemittel mit seitenständigen Amin-funktionellen Gruppen, Carbonsäure-funktionellen Gruppen und einer T_{g} von mehr als 0°C, wobei die seitenständigen Amin-funktionellen Gruppen und die Carbonsäuregruppen Teil des gleichen Polymers sind, einführbar durch das Copolymerisieren Amin-substituierter Monomere und Carbonsäure-Monomere, oder ein Polymerbindemittel, umfassend ein Polymer mit Carbonsäure-funktionellen Gruppen und einer T_{g} von mehr als 0°C und ein Polymer mit seitenständigen Amin-funktionellen Gruppen und einer T_{g} von mehr als 0°C,
(b) eine Menge flüchtiger Base, ausreichend, um den pH der Zusammensetzung auf einen Punkt zu heben, an dem alle Amin-funktionellen Gruppen in einem nicht-ionischen Zustand vorliegen,
(c) Parfum und
(d) Wasser,
wobei die Zusammensetzung weniger als 20% organische Lösungsmittel und weniger als 10% von Ammoniak und Wasser verschiedenes anorganisches Material enthält.

7. Wässrige Zusammensetzung nach Anspruch 6, in welcher die Zusammensetzung weniger als 12% organische Lösungsmittel und weniger als 5% VOC-Lösungsmittel enthält.

8. Wässrige Zusammensetzung nach Anspruch 7, weiter umfassend mindestens ein Polyurethanbindemittel.

9. Wässrige Zusammensetzung nach Anspruch 6, in welcher das Polymerbindemittel umfasst:
(a) ein Polymer mit seitenständigen Amin-funktionellen Gruppen und einer T_{g} von mehr als 0°C und
(b) ein Polymer mit Carbonsäure-funktionellen Gruppen und einer T_{g} von mehr als 0°C.

10. Wässrige Zusammensetzung nach Anspruch 9, weiter umfassend mindestens ein Polyurethanbindemittel.

## Revendications

1. Procédé d'enduction d'ongles humains avec un film de polymère ; ledit procédé comprenant les étapes consistant :
(a) à appliquer aux ongles humains une composition comprenant : (i) un liant polymère ayant des groupes fonctionnels amine pendants, des groupes fonctionnels acide carboxylique et une Tg supérieure à 0 °C, dans lequel les groupes fonctionnels amine pendants et les groupes carboxyliques font partie du même polymère, pouvant être introduits en copolymérisant des monomères substitués par une amine et des monomères d'acide carboxylique, ou un liant polymère comprenant un polymère ayant des groupes fonctionnels acide carboxylique et une Tg supérieure à 0 °C et un polymère ayant des groupes fonctionnels amine pendants et une Tg supérieure à 0 °C ; (ii) une quantité de base volatile suffisante pour élever le pH de la composition jusqu'à un point où tous les groupes fonctionnels amine sont dans un état non ionique ; et (iii) de l'eau ; dans lequel la composition contient moins de 20 % de solvants organiques ; et
(b) à laisser la composition sécher jusqu'à ce que le film de polymère se forme.

2. Procédé selon la revendication 1, dans lequel la composition aqueuse contient moins de 12 % de solvants organiques, moins de 5 % de solvants de type COV, et moins de 10 % de matière inorganique.

3. Procédé selon la revendication 2, comprenant en outre de 1 à 10 % d'une cire.

4. Procédé selon la revendication 1, dans lequel ledit liant polymère comprend :
(a) un polymère ayant des groupes fonctionnels amine pendants et une Tg supérieure à 0 °C ; et
(b) un polymère ayant des groupes fonctionnels acide carboxylique et une Tg supérieure à 0 °C.

5. Procédé selon la revendication 4, dans lequel la composition aqueuse contient moins de 12 % de solvants organiques, moins de 5 % de solvants de type COV, et comprenant en outre de 1 à 10 % d'une cire.

6. Composition aqueuse appropriée pour une utilisation en tant que vernis à ongles comprenant :
(a) un liant polymère ayant des groupes fonctionnels amine pendants, des groupes fonctionnels acide carboxylique et une Tg supérieure à 0 °C, dans lequel les groupes fonctionnels amine pendants et les groupes carboxyliques font partie du même polymère, pouvant être introduits en copolymérisant des monomères substitués par une amine et des monomères d'acide carboxylique, ou un liant polymère comprenant un polymère ayant des groupes fonctionnels acide carboxylique et une Tg supérieure à 0 °C et un polymère ayant des groupes fonctionnels amine pendants et une Tg supérieure à 0 °C ;
(b) une quantité de base volatile suffisante pour élever le pH de la composition jusqu'à un point où tous les groupes fonctionnels amine sont dans un état non ionique ;
(c) du parfum ; et
(d) de l'eau ;
dans laquelle la composition contient moins de 20 % de solvants organiques et moins de 10 % de matière inorganique qui est différente de l'ammoniaque et de l'eau.

7. Composition aqueuse selon la revendication 6, dans laquelle la composition contient moins de 12 % de solvants organiques et moins de 5 % de solvants de type COV.

8. Composition aqueuse selon la revendication 7, comprenant en outre au moins un liant à base de polyuréthanne.

9. Composition aqueuse selon la revendication 6, dans laquelle ledit liant polymère comprend :
(a) un polymère ayant des groupes fonctionnels amine pendants et une Tg supérieure à 0 °C ; et
(b) un polymère ayant des groupes fonctionnels acide carboxylique et une Tg supérieure à 0 °C.

10. Composition aqueuse selon la revendication 9, comprenant en outre au moins un liant à base de polyuréthanne.
